# EUROPEAN PATENT APPLICATION

(11) **EP 4 238 548 A1**
(43) Date of publication of application: **06.09.2023**
(21) Application number: 22159537.4
(22) Date of filing: 01.03.2022
(51) Int. Cl.: A61K 8/46, A61Q 19/08

(54) **NOVEL USE OF 4-AMIDINO BENZYLAMINES**

(71) Applicant: DSM IP Assets B.V., 6411 TE Heerlen (NL)
(72) Inventor: BUDEL, Leithe, 4303 Kaiseraugst (CH)
(74) Representative: Berg, Katja

(57) **Abstract**

The present invention relates to the use of 4-amidino benzylamines, especially Amidinobenzyl Benzylsulfonyl D-Seryl Homophenylalaninamide Acetate, as anti-ageing active ingredient as well as the use thereof in topical anti-ageing compositions.

## Description

The present invention relates to the use of certain 4-amidino benzylamines as anti-ageing active ingredient as well as the use thereof in topical anti-ageing compositions.

The human skin is the biggest organ of the human body, and it is the barrier that separates the body from the outer environment. Human skin is made up of several layers that are classified as epidermis, dermis and subcutaneous tissue. The epidermis is the outermost layer of the skin and is mainly formed of keratinocytes comprising about 90% of the epidermis and melanocytes which are found at the base of the epidermis and make melanin. The main function of epidermis is to form a permeability barrier against environmental challenges, such as UV radiation, heat, chemicals, pollution, and pathogens, such as bacteria, fungi, parasites, and viruses. It also protects the body from uncontrolled water evaporation from inside out, maintaining the hydration balance and skin metabolism.

The dermis is the second layer of skin. In the dermis, the most abundant cell type are the dermal fibroblasts, which are responsible of generating the connective tissue by producing extracellular matrix (ECM). Said ECM is a three-dimensional network consisting of extracellular macromolecules and minerals, such as collagen, enzymes, glycoproteins and hydroxyapatite. The ECM imparts strength and integrity to the skin.

As a major structural protein of the extracellular matrix (ECM), type I collagen is secreted by fibroblasts, forming more than 90% of the dry weight of the dermis. It is produced as procollagen which is then processed to mature collagen.

The aging process is a dynamic and unchangeable phenomenon which affects all systems in the body. It is known that endogenous (age-related) or exogenous (chronic light exposure, heat, pollution, chemicals, toxins) aging leads to an irreversible degeneration of tissues, in particular of skin. In skin, visible signs of ageing are inter alia fine-lines, wrinkles and sagging. Modifications in collagen, the most important protein of the connective tissue, are the main factors for these anatomic changes. Even though collagen is constantly produced and recycled, with increasing age the rate of synthesis of collagen significantly reduces thus leading to the visible manifestations of skin ageing.

Thus, there is an ongoing need for ingredients which are able to increase collagen production and are thus able to counteract skin-ageing and the visible signs associated therewith.

Surprisingly it has now been found that certain 4-amidino benzylamine are able to significantly increase collagen synthesis in human dermal fibroblasts.

Thus, in a first embodiment, the present invention concerns a non-therapeutic use of a compound of formula (I) 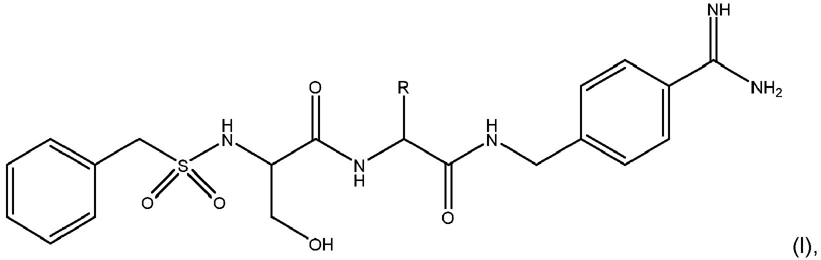 wherein R is a C₁ to C₆ linear or branched aliphatic hydrocarbon chain optionally substituted with a C₆ to C₁₀ aromatic group, for preventing, reducing or treating skin aging in a subject (in need thereof).

In another aspect, the invention concerns a compound of formula (I) as outlined above for use in prevention, reduction or treatment of skin aging in a subject.

In a further aspect, the invention concerns a method of preventing, reducing or treating skin aging in a subject, said method comprising administering a compound of formula (I) as outlined above to said subject, in particular to a skin area in need thereof such as e.g. to facial skin, skin of the hands and photoaged skin.

Collagen is responsible for skin strength and structure; collagen homeostasis in the deeper part of the skin is maintained by balancing the degradative and synthetic pathways and a skewed balance between collagen production and degradation can lead to skin aging.

It has been found in accordance with the present invention, that compounds of formula (I) as outlined above increase type I collagen in human dermal fibroblasts and are thus able to maintain skin homeostasis, in particular in ageing and/or aged skin.

Thus, the present invention also relates to the use of a compound of formula (I) as outlined above for inreasing (type I) collagen production in fibroblasts and accordingly in skin (compared to an untreated control).

The invention also relates to a use of a compound of formula (I) as outlined above for increasing the expression of (type I) collagen in fibroblasts (compared to an untreated control).

Preferably, in all embodiments of the present invention, the fibroblasts are human fibroblasts, most preferably dermal human fibroblasts.

In all the embodiments of the present invention, the compound of formula (I) is characterized in that R is a C₁ to C₆ linear or branched aliphatic hydrocarbon chain optionally substituted with a C₆ to C₁₀ aromatic group, preferably, R is a C₁ to C₃ linear aliphatic hydrocarbon chain substituted with a C₆ to C₈ aromatic group, more preferably, R is a C₁ to C₃ linear aliphatic hydrocarbon chain substituted with a, preferably terminal, phenyl group (such as in particular phenylmethyl-, phenylethyl- and phenylpropyl-), most preferably R is -CH₂-CH₂-C₆H₅ meaning that the compound of formula (I) is benzylsulfonyl-Ser-hPhe-4-amidinobenzylamide (formula (II)).

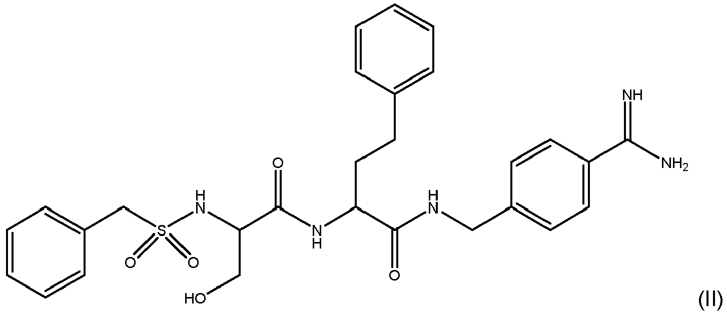

In all embodiments of the present invention, the compound of formula (I) respectively formula (II) can be in any stereo chemical configuration.

It is however preferred, in all embodiments of the present invention the compounds of formula (I) and (II) are compounds of formula (Ia) or (IIa).

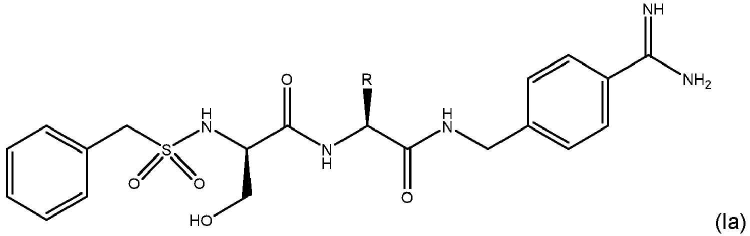

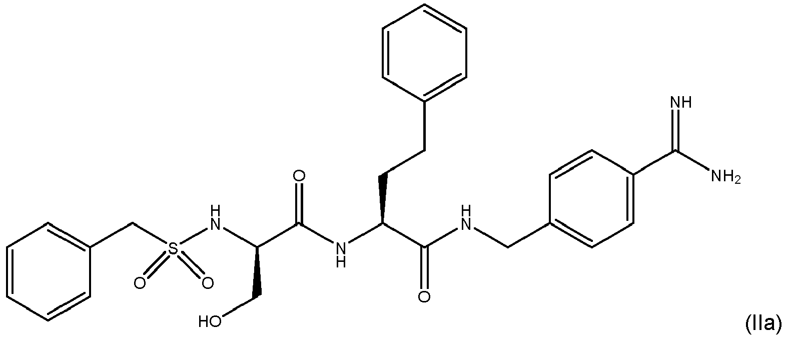

Preferably, in all embodiments of the present invention, the compounds of formula (I) and (Ia) as well as (II) and (IIa) are used in the form of salts thereof, preferably with mineral acids, preferably as hydrochlorides or as salts with suitable organic acids or sulfates,e.g. as acetates or sulfates. Preferred organic acids salts for the compounds of formula (I), (Ia), (II) and (IIa) are selected from acetic acid, formic acid, methylsulfonic acid, succinic acid, malic acid, trifluoroacetic acid, most preferred being the respective acetate salts.

Most preferred in all embodiments of the present invention is the use of Amidinobenzyl Benzylsulfonyl D-Seryl Homophenylalaninamide Acetate (CAS No: 1393815-16-1), which is commercially available in the form of SYN^{®}-UP at DSM Nutritional Products Ltd, Kaiseraugst. SYN^{®}-UP is a mixture of Amidinobenzyl Benzylsulfonyl D-Seryl Homophenylalaninamide Acetate (0.1-1 wt-%), water (25-50 wt.%), citric acid (0.1-1 wt.-%), sodium hydroxide (less than 0.1 wt.-%, for pH adjustment to 2.6-3.6) and glycerin (up to 100%). In all embodiments of the present invention, the use of said mixture (in the following also referred to as SYN^{®}-UP mixture) is most preferred.

The term 'subject', as used herein, means an animal having a skin that separates and defines the animal inside from the outside. Preferably, the subject is a human.

In one embodiment of the present invention the subject may be female. In another embodiment the subject may be male. In a further embodiment the subject may be with a non-binary gender.

In a preferred embodiment the subject is not a child. The term 'child' as used herein means a human Io 7 years of age or younger. Thus, in all embodiments of the present invention, the subject is a human that is 8 years of age or older, preferably the subject is a human being 20 years or older, preferably 30 years or older, even more preferably 40 years or older such as most preferably 50 years or older.

In a further embodiment, the prevention, reduction or treatment of the skin aging in a subject is attained by increasing or maintaining the production of type I collagen or slowing down the reduction of type I collagen in the skin and/or maintaining collagen metabolism or slowing down the reduction of the collagen metabolism in the skin.

In a further embodiment, the present invention relates to the prevention, reduction or treatment of skin aging caused by intrinsic and/ or extrinsic factors, such as, but not limited to sun, sunburn, collagen damage, senescence, infrared radiation, heat, hormonal reasons, malnutrition, temperature, tobacco smoking, stress, sleep deprivation, pollution, or alcohol consumption.

The term 'prevention, reducing or treating skin aging' includes the prevention, reduction or treatment of fine lines, wrinkles, crow's feet, sagging, skin thinning and/ or rough skin texture as well as improving skin elasticity or skin firmness without being limited thereto.

The term 'prevention, reducing or treating skin aging' also encompass smoothening of wrinkles and fine lines as well as decreasing their volume and depth in a person in need thereof.

In all embodiments of the present invention preferably the use is non-therapeutic, i.e. a cosmetic use intended for beautifying the skin.

In all embodiments of the present invention, the compounds of formula (I), (Ia), (II) and (IIa) such as in particular Amidinobenzyl Benzylsulfonyl D-Seryl Homophenylalaninamide Acetate or the SYN^{®}-UP mixture are preferably administered in the form of a cosmetic or dermatological composition.

The term 'cosmetic composition' as used herein refers to cosmetic compositions which are used to treat, care for or improve the appearance of the skin and/or the scalp, preferably the skin, most preferably facial skin and/ or the skin of the hands.

The term 'dermatological composition' as used herein refers to compositions which are used to be applied to skin.

The total amount of the compounds of formula (I), (Ia), (II) and (IIa) such as in particular of Amidinobenzyl Benzylsulfonyl D-Seryl Homophenylalaninamide Acetate in the compositions according to the present invention is preferably selected in the range from 0.00001 to 0.1 wt.-%, more preferably in the range from 0.0001 to 0.06 wt.-%, most preferably in the range from 0.0005 to 0.03 wt.-%, based on the total weight of the composition. Further suitable ranges are selected in the range of 0.00001 to 0.01 wt.-%, more preferably in the range from 0.0001 to 0.005 wt.-%, most preferably in the range from 0.0005 to 0.002 wt.-%,

The total amount of the SYN^{®}-UP mixture in the compositions according to the present invention is preferably selected in the range from 0.01 to 10 wt.-%, more preferably in the range from 0.1 to 6 wt.-%, most preferably in the range from 0.5 to 3 wt.-%, based on the total weight of the composition.

If nothing else is stated in this specification any given parts and percentages are per weight and based on the total weight of the composition.

In a particular embodiment, the compounds of formula (I), (Ia), (II) and (IIa) such as in particular of Amidinobenzyl Benzylsulfonyl D-Seryl Homophenylalaninamide Acetate are used in the compositions according to the present invention either in the form of the SYN^{®}-UP mixture or in admixture with glycerin, water and/ or citric acid.

In a particular embodiment, the compositions according to the present invention are cosmetic compositions intended to be topically applied to mammalian keratinous tissue such as in particular to human skin or the human scalp. Such compositions are also called dermatological compositions. Thus, preferably in all embodiments of the present invention the cosmetic compositions are topical cosmetic (i.e. dermatological) compositions with all the definitions and preferences as given herein.

The topical cosmetic compositions according to the present invention may be leave-on or rinse-off compositions, and include any product applied to a human body, primarily for improving appearance or general aesthetics. Preferably the cosmetic compositions of the present invention are leave-on compositions.

It is well understood that the cosmetic compositions according to the invention intended for topical application comprise a physiologically acceptable medium, i.e. a medium compatible with keratinous substances, such as the skin, mucous membranes, and keratinous fibers. In particular, the physiologically acceptable medium is a cosmetically acceptable carrier. In all embodiments of the present invention, it is preferred that the carrier comprises water.

The term 'cosmetically acceptable carrier' (also referred to herein as carrier) refers to all vehicles/ carriers conventionally used in cosmetic compositions, i.e. which are suitable for topical application to the keratinous tissue, have good aesthetic properties, are compatible with the actives present in the composition, and will not cause any unreasonable safety or toxicity concerns. Such carriers are well-known to one of ordinary skill in the art and can include one or more compatible liquid(s) or solid filler diluent(s), excipient(s), additive(s) or vehicle(s) which are suitable for application to skin.

The exact amount of carrier will depend upon the actual level of the active ingredients and of any other optional ingredients that one of ordinary skill in the art would classify as distinct from the carrier (e.g., other active ingredients).

In an advantageous embodiment, the cosmetic compositions according to the present invention comprise from about 50% to about 99%, preferably from about 60% to about 98%, more preferably from about 70% to about 98%, such as in particular from about 80% to about 95% of a carrier, based on the total weight of the cosmetic composition.

In a particular advantageous embodiment, the carrier in the cosmetic compositions according to the present invention consists of at least 30 wt. %, more preferably of at least 40 wt.-%, most preferably of at least 45 wt.-% of water, such as in particular of 50 to 90 wt.-% of water.

In a particular advantageous embodiment, the cosmetic compositions according to the present invention comprise glycerin in an amount of 0.005 to 10 wt.-%, preferably in the range of 0.05 to 5 wt.-%, most preferably in the range of 0.25 to 4 wt.-%, such as in the range of 0.25 to 3 wt.-%, based on the total weight of the composition.

The cosmetic compositions in accordance with the invention can be in the form of a liquid, lotion, a thickened lotion, a gel, a cream, a milk, an ointment, a paste, a powder, a make-up, or a solid tube stick and can be optionally be packaged as an aerosol and can be provided in the form of a mousse such as an aerosol mousse, a foam or a spray foam, a spray, a stick.

Preferably the compounds of formula (I), (Ia), (II) and (IIa) such as in particular of Amidinobenzyl Benzylsulfonyl D-Seryl Homophenylalaninamide Acetate, preferably in the form of the SYN^{®}-UP mixture according to the present invention are formulated into lotions, creams, gels, and tonics. These product forms may be used for a number of applications, including, but not limited to, hand and body lotions, (facial) anti-ageing preparations, make-ups including foundations, sunscreens, sunless tanners and the like. Any additional components required to formulate such products vary with product type and can be routinely chosen by one skilled in the art.

If the cosmetic compositions of the present invention are formulated as an aerosol and applied to the skin as a spray-on product, a propellant is added to the composition.

The cosmetic compositions according to the present invention can be prepared by conventional methods in the art such as e.g. by admixing the compounds of formula (I), (Ia), (II) and (IIa) such as in particular of Amidinobenzyl Benzylsulfonyl D-Seryl Homophenylalaninamide Acetate or the SYN^{®}-UP mixture with all the definitions and preferences given herein with the cosmetically acceptable carrier.

The cosmetic composition may comprise further ingredients, which may form part of the carrier. Such ingredients are particularly surfactants, emulsifiers, thickeners, and oils. Such suitable surfactants, emulsifiers, thickeners, and oils are well known to a person skilled in the art.

The cosmetic compositions of the invention (including the carrier) may comprise further conventional (cosmetic) adjuvants and additives, such as preservatives/antioxidants, fatty substances/oils, water, organic solvents, silicones, thickeners, softeners, emulsifiers, antifoaming agents, aesthetic components such as fragrances, surfactants, fillers, anionic, cationic, non-ionic or amphoteric polymers or mixtures thereof, propellants, acidifying or basifying agents, dyes, colourings/colorants, abrasives, absorbents, chelating agents and/ or sequestering agents, essential oils, skin sensates, astringents, pigments or any other ingredients usually formulated into such compositions.

In accordance with the present invention, the compositions according to the invention may also comprise further cosmetically active ingredients conventionally used in cosmetic compositions. Exemplary active ingredients encompass skin lightening agents; UV-filters, agents for the treatment of hyperpigmentation; agents for the prevention or reduction of inflammation; firming, moisturizing, soothing, and/ or energizing agents as well as agents to improve elasticity and skin barrier.

If nothing else is stated, the excipients, additives, diluents, etc. mentioned in the following are suitable for the compositions according to the present invention. The necessary amounts of the cosmetic and dermatological adjuvants and additives can, based on the desired product, easily be determined by the skilled person.

The additional ingredients can either be added to the oily phase, the aqueous phase or separately as deemed appropriate. The mode of addition can easily be adapted by a person skilled in the art.

Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

The cosmetic compositions according to the present invention are in particular skin care preparations, or functional (anti-ageing) preparations.

Examples of skin care preparations are, in particular, light protective preparations (sun care preparations), anti-ageing preparations, preparations for the treatment of photo-ageing, body oils, body lotions, body gels, treatment creams, skin protection ointments, moisturizing preparations such as moisturizing gels or moisturizing sprays, face and/or body moisturizers, as well as skin lightening preparations.

Examples of functional preparations are cosmetic compositions containing active ingredients such as hormone preparations, vitamin preparations, vegetable extract preparations, anti-ageing preparations, and/or antimicrobial (antibacterial or antifungal) preparations without being limited thereto.

The cosmetic compositions according to the present invention can be in a wide variety of forms. Non limiting examples include simple solutions (e.g. aqueous, organic solvent, or oil based), emulsion or micro emulsion (in particular of oil-in-water (O/W) or water-in-oil (W/O) type, silicone-in-water (Si/W) or water-in-silicone (W/Si) type, PIT-emulsion, multiple emulsion (e.g. of oil-in-water-in oil (O/W/O) or water-in-oil-in-water (W/O/W) type) or pickering emulsions), as well as solid forms (e.g. hydrogels, alcoholic gels, lipogels, sticks, flowable solids, or amorphous materials).

These product forms may be used for a number of applications, including, but not limited to, gels, creams, ointments, lotions, serums, powder, aerosol sprays or two component dispensing systems.

In a preferred embodiment, the cosmetic compositions according to the present invention are emulsions and/or gels. Even more preferably, the cosmetic compositions are emulsions which contain an oily phase and an aqueous phase such as in particular O/W, W/O, Si/W, W/Si, O/W/O, W/O/W multiple or a pickering emulsions.

The amount of the oily phase (i.e. the phase containing all oils and fats including the polar oils) present in such emulsions such as in particular O/W, W/O, Si/W, W/Si, O/W/O, W/O/W multiple or a pickering emulsions is preferably at least 10 wt.-%, such as in the range from 10 to 60 wt.-%, preferably in the range from 15 to 50 wt.-%, most preferably in the range from 15 to 40 wt.-%, based on the total weight of the composition.

The oil phase according to the invention preferably comprises oils selected from butylenglykoldicaprylat/-dicaprat, propylenglykoldicaprylat/-dicaprat, dicaprylylether, C₁₂₋₁₅-Alkylbenzoat, C₁₈₋₃₈-fatty acid triglyceride, dibutyladipate, cyclomethicone, dimethicone, 2-phenylethylbenzoat, isopropyl lauroyl sarkosinate, caprylic/ capric triglyceride as well as mixtures thereof.

The amount of the aqueous phase present in such emulsions is preferably at least 20 wt.-%, such as in the range from 20 to 90 wt.-%, preferably in the range from 30 to 80 wt.-%, most preferably in the range from 30 to 70 wt.-%, based on the total weight of the composition.

Advantageously in all emulsions of the present invention the ratio of oily phase to aqueous phase is selected in the range of 40:60 to 30 to 70.

In one particular advantageous embodiment, the cosmetic compositions according to the present invention are in the form of an oil-in-water (O/W) emulsion comprising an oily phase dispersed in an aqueous phase in the presence of an O/W emulsifier. The preparation of such O/W emulsions is well known to a person skilled in the art.

If the cosmetic composition according to the invention is an O/W emulsion, then it contains advantageously at least one O/W- or Si/W-emulsifier selected from the list of, glyceryl stearate citrate, glyceryl stearate SE (self-emulsifying), stearic acid, salts of stearic acid, polyglyceryl-3-methylglycosedistearate. Further suitable emulsifiers are phosphate esters and the salts thereof such as cetyl phosphate (e.g. as Amphisol^{®} A from DSM Nutritional Products Ltd.), diethanolamine cetyl phosphate (e.g. as Amphisol^{®} DEA from DSM Nutritional Products Ltd.), potassium cetyl phosphate (e.g. as Amphisol^{®} K from DSM Nutritional Products Ltd.), sodium cetearylsulfate, sodium glyceryl oleate phosphate, hydrogenated vegetable glycerides phosphate and mixtures thereof. Further suitable emulsifiers are sorbitan oleate, sorbitan sesquioleate, sorbitan isostearate, sorbitan trioleate, cetearyl glucoside, lauryl glucoside, decyl glucoside, sodium stearoyl glutamate, sucrose polystearate and hydrated polyisobutene. Furthermore, one or more synthetic polymers may be used as an emulsifier. For example, PVP eicosene copolymer, acrylates/C10-30 alkyl acrylate crosspolymer, and mixtures thereof.

The at least one O/W, respectively Si/W emulsifier is preferably used in an amount of 0.5 to 10 wt. %, in particular in the range of 0.5 to 6 wt.-%, such as more in particular in the range of 0.5 to 5 wt.-%, such as most in particular in the range of 1 to 4 wt.-%, based on the total weight of the cosmetic composition.

Particular suitable O/W emulsifiers to be used in the cosmetic compositions according to the invention encompass phosphate ester emulsifiers such as advantageously 8-10 alkyl ethyl phosphate, C9-15 alkyl phosphate, ceteareth-2 phosphate, ceteareth-5 phosphate, ceteth-8 phosphate, ceteth-10 phosphate, cetyl phosphate, C6-10 pareth-4 phosphate, C12-15 pareth-2 phosphate, C12-15 pareth-3 phosphate, DEA-ceteareth-2 phosphate, DEA-cetyl phosphate, DEA-oleth-3 phosphate, potassium cetyl phosphate, deceth-4 phosphate, deceth-6 phosphate and trilaureth-4 phosphate.

A particular suitable O/W emulsifier to be used in the cosmetic compositions according to the invention is potassium cetyl phosphate e.g. commercially available as Amphisol^{®} K at DSM Nutritional Products Ltd Kaiseraugst.

Another particular suitable class of O/W emulsifiers are non-ionic self-emulsifying systems derived from olive oil e.g. known as (INCI Name) cetearyl olivate and sorbitan olivate (chemical composition: sorbitan ester and cetearyl ester of olive oil fatty acids) sold under the tradename OLIVEM 1000.

In one particular embodiment, the invention relates to cosmetic compositions with all the definitions and preferences given herein in the form of O/W emulsions comprising an oily phase dispersed in an aqueous phase in the presence of an O/W emulsifier wherein the O/W emulsifier is potassium cetyl phosphate. The amount of oily phase in such O/W emulsions is preferably at least 10 wt.-%, more preferably in the range of 10 to 60 wt.-%, most preferably in the range of 15 to 50 wt.-%, such as in the range of 15 to 40 wt.-%, based on the total weight of the composition.

Preferably, the cosmetic compositions according to the invention further comprise at least one fatty alcohol (co-emulsifier), such as in particular cetyl alcohol, cetearyl alcohol and/ or behenyl alcohol. The total amount of one or several fatty alcohols on the topical compositions according to the invention is preferably selected in the range of about 0.1 to 10.0 wt.-%, in particular in the range of about 0.5 to 6.0 wt.-% with respect to the total weight of the topical composition.

Preferably, the topical compositions according to the invention comprise a thickener in particular if the topical composition is in the form of an emulsion to assist in making the consistency of a product suitable. Preferred thickeners are aluminiumsilicates, xanthan gum, hydroxypropylmethylcellulose, hydroxyethylcellulose, polyacrylates such as carbopole^{®} (e.g. Carbopole 980, 981, 1382, 2984, 5984) or mixtures thereof. Further preferred thickeners encompass acrylate/C₁₀₋₃₀ alkyl acrylate copolymers (such as e.g. Pemulen TR 1, Pemulen TR 2, Carbopol 1328 by. NOVEON) as well as Aristoflex AVC (INCI: Ammonium Acryloyldimethyltaurate/VP Copolymer).

The cosmetic compositions according to the present invention advantageously comprise a preservative. When present, the preservative is preferably used in an amount of 0.1 to 2 wt.-%, more preferably in an amount of 0.5 to 1.5 wt.-%, based on the total weight of the composition.

The cosmetic compositions according to the invention in general have a pH in the range of 3 to 10, preferably a pH in the range of 4 to 8 and most preferably a pH in the range of 4 to 7.5 such as in the range of 5 to 6.5. The pH can easily be adjusted as desired with suitable acids, such as e.g. citric acid, or bases, such as sodium hydroxide (e.g. as aqueous solution), triethanolamine (TEA Care), Tromethamine (Trizma Base) and Aminomethyl Propanol (AMP-Ultra PC 2000), according to standard methods in the art.

The amount of the cosmetic composition to be applied to the skin is not critical and can easily be adjusted by a person skilled in the art. Preferably the amount is selected in the range of 0.1 to 3 mg/cm² skin, such as preferably in the range of 0.1 to 2 mg/cm² skin and most preferably in the range of 0.5 to 2 mg/cm² skin.

The following examples are provided to further illustrate the compositions and effects of the present invention. These examples are illustrative only and are not intended to limit the scope of the invention in any way.

### Examples

### Short description

Amidinobenzyl Benzylsulfonyl D-Seryl Homophenylalaninamide Acetate (ABSHA) was evaluated for its effect on collagen type 1. This was done using an in vitro dermal fibroblast collagen assay. It was found that Amidinobenzyl Benzylsulfonyl D-Seryl Homophenylalaninamide Acetate increases collagen expression.

### Methodology

Normal human dermal fibroblasts were seeded in cell culture medium (Dulbecco's Modified Eagle Medium supplemented with 10% serum and 1% antibiotics) and allowed to adhere in a humidified cell incubator maintaining 37°C and 5% CO₂. Afterwards, the cells were starved with culture medium without serum for 16-24 hours to synchronize the cells their cell cycle. The starvation medium was removed and cell culture medium + SYN^{®}-UP at various concentrations was added to the cells. The cells were treated for 72 hours. The cells were then fixated and immunolabeled with antibodies specific for collagen 1. These labeled cells were analyzed using flow cytometry and compared to the untreated ones (medium without SYN^{®}-UP).

### Results

**Table 1. Normal human dermal fibroblasts were treated for 72 hours with several concentrations of Amidinobenzyl Benzylsulfonyl D-Seryl Homophenylalaninamide Acetate (ABSHA), which resulted in the increase of collagen type 1 proteins. Data are normalized to the untreated control (100%) and expressed as the mean ± SD (n = 4).**

| **Condition** | **Collagen 1 expression relative to untreated (%)** | **Standard deviation (n=4)** | **P-value (compared to untreated)** |
|---|---|---|---|
| Untreated | 100 | 4.2 | - |
| ABSHA 1 µM | 144 | 13.8 | 0.02333 |
| ABSHA 5 µM | 160 | 9.6 | 0.00018 |
| ABSHA µM | 188 | 8.5 | 0.00000 |
| ABSHA 25 µM | 185 | 11.8 | 0.00003 |

## Claims

1. Non-therapeutic use of a compound of formula (I) 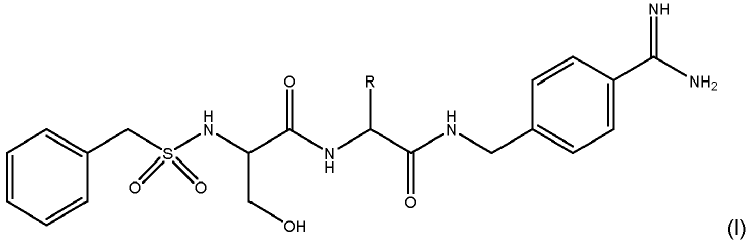
wherein R is a C₁ to C₆ linear or branched aliphatic hydrocarbon chain optionally substituted with a C₆ to C₁₀ aromatic group
for preventing, reducing or treating skin aging in a subject.

2. The non-therapeutic use according to claim 1, wherein R is a C₁ to C₃ linear aliphatic hydrocarbon chain substituted with a C₆ to C₈ aromatic group, preferably R is a C₁ to C₃ linear aliphatic hydrocarbon chain substituted with a phenyl group, most preferably R is a phenylmethyl-, a phenylethyl- or a phenylpropyl- group.

3. The non-therapeutic use according to claim 1 and/ or 2, wherein R is -CH₂-CH₂-C₆H₅.

4. The non-therapeutic use according to any one or more of the preceding claims, wherein the compound of formula (I) is Amidinobenzyl Benzylsulfonyl D-Seryl Homophenylalaninamide Acetate.

5. The non-therapeutic use according to any one or more of the preceding claims, wherein the compound of formula (I) increases or maintains production of type I collagen or slows down the reduction of type I collagen in the skin.

6. The non-therapeutic use according to any one or more of the preceding claims, wherein the use is in form of a cosmetic or dermatological composition comprising the compound of formula (I).

7. The non-therapeutic use according to claim 6, wherein the amount of the compound of formula (I) in the composition is selected in the range from 0.00001 to 0.1 wt.-%, more preferably in the range from 0.0001 to 0.06 wt.-%, most preferably in the range from 0.0005 to 0.03 wt.-%, based on the total weight of the composition based on the total weight of the composition.

8. The non-therapeutic use according to claim 6 and/ or 7, wherein the composition is a leave-on compositions.

9. The non-therapeutic use according to any one or more of claims 6 to 8, wherein the composition comprises a carrier consisting of at least 30 wt. %, more preferably of at least 40 wt.-%, most preferably of at least 45 wt.-% of water, such as in particular of 50 to 90 wt.-% of water.

10. The non-therapeutic use according to any one or more of claims 6 to 9, wherein, the composition is an O/W emulsion comprising an oily phase dispersed in an aqueous phase.

11. A method for preventing, reducing or treating skin aging in a subject, preferably in a subject aged 30 years or older, preferably 40 years or older most preferably 50 years or older, said method comprising the step of administering to a skin area in need thereof a cosmetic composition comprising a compound of formula (I) 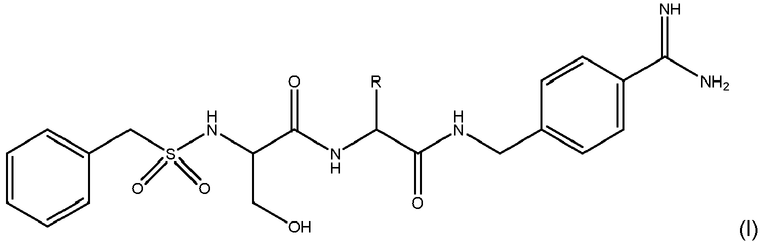 wherein R is a C₁ to C₆ linear or branched aliphatic hydrocarbon chain optionally substituted with a C₆ to C₁₀ aromatic group.

12. The method according to claim 11, wherein R is a C₁ to C₃ linear aliphatic hydrocarbon chain substituted with a C₆ to C₈ aromatic group, preferably R is a C₁ to C₃ linear aliphatic hydrocarbon chain substituted with a phenyl group, most preferably R is a phenylmethyl-, a phenylethyl- or a phenylpropyl- group.

13. The method according to claim 11 and/or 12, wherein the amount of the compound of formula (I) in the composition is selected in the range from 0.00001 to 0.1 wt.-%, more preferably in the range from 0.0001 to 0.06 wt.-%, most preferably in the range from 0.0005 to 0.03 wt.-%, based on the total weight of the composition.

14. The method according to anyone or more of claims 11 to 13, wherein the amount of the composition to be applied to the skin is selected in the range of 0.1 to 3 mg/cm² skin, such as preferably in the range of 0.1 to 2 mg/cm² skin and most preferably in the range of 0.5 to 2 mg/cm² skin.

15. Use of a compound of formula (I) 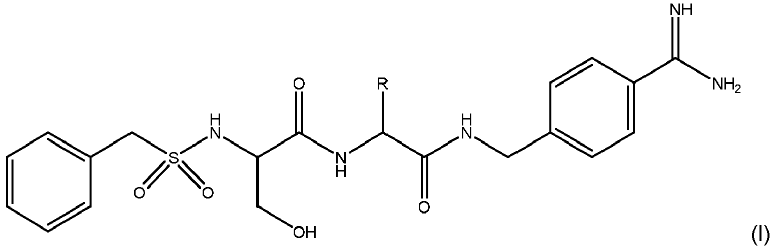 wherein R is a C₁ to C₆ linear or branched aliphatic hydrocarbon chain optionally substituted with a C₆ to C₁₀ aromatic group
for increasing the expression of type I collagen in human fibroblasts.
